# EUROPÄISCHE PATENTSCHRIFT

(11) **EP 0 357 558 B1**
(45) Veröffentlichungstag und Bekanntmachung des Hinweises auf die Patenterteilung: **02.02.1994**
(21) Anmeldenummer: 89810630.7
(22) Anmeldetag: 24.08.1989
(51) Int. Cl.: C07D 209/76, C08G 73/12

(54) **Bisimide der Allyl- oder Methallylbicyclo[2,2,1]hept-5-en-2,3-dicarbonsäure**
Bisimides of allyl-substituted or methallyl-substituted bicyclo[2.2.1]hept-5-ene-2,3-dicarboxylic acid
Bisimides de l'acide allyl- ou méthallylbicyclo-[2.2.1]heptène-5 dicarboxylique-2,3

(30) Priorität: 02.09.1988 CH 3281/88
(43) Veröffentlichungstag der Anmeldung: 07.03.1990
(73) Patentinhaber: CIBA-GEIGY AG, 4002 Basel (CH)
(72) Erfinder: Stockinger, Friedrich, CH-1784 Courtepin (CH); Kramer, Andreas, Dr., CH-3186 Düdingen (CH)

(56) Entgegenhaltungen:
- EP-A- 0 105 024
- EP-A- 0 152 372
- EP-A- 0 175 648
- EP-A- 0 192 480
- EP-A- 0 195 402
- CH-A- 621 768
- US-A- 4 535 170

## Beschreibung

Die vorliegende Erfindung betrifft ausgewählte Bisimide der Allyl- oder Methallyl-bicyclo[2.2.1]hept-5-en-2,3-dicarbonsäure, ein Verfahren zu deren Herstellung, ausgewählte Zwischenprodukte und die durch Härten dieser Bisimide erhältlichen vernetzten Produkte.

In der EP-A-105,024 sind unter anderem Bisimide auf Basis von Allyl- oder Methallyl-bicyclo[2.2.1]hept-5-en-2,3-di-carbonsäure und Alkylendiaminen oder zweikernigen carbocyclisch-aromatischen Diaminen beschrieben. Die Verbindungen sind hitzehärtbar und eignen sich beispielsweise zur Herstellung von faserverstärkten Kunststoffen.

Aus der EP-A-152,372 sind unter anderem Bisimide auf Basis von Allyl- oder Methallyl-methyl-bicyclo[2.2.1]hept-5-en-2,3-dicarbonsäureanhydrid und den oben genannten Diaminen bekannt. Diese Verbindungen eignen sich ebenfalls zur Herstellung von Matrixharzen für Verbundwerkstoffe.

Ferner sind aus der EP-A-175,648 Kombinationen der obengenannten Bisimidderivate mit Polymaleinimiden bekannt. Die gehärteten Produkte aus diesen Kombinationen zeichnen sich durch gute mechanische Eigenschaften aus, wie gute Biege-, Zugscher- oder interlaminare Scherfestigkeit, und ferner durch eine geringe Sprödigkeit und durch hohe Glasumwandlungstemperaturen.

Es wurden jetzt Bisimide auf Basis von Allyl- oder Methallyl-bicyclo-[2.2.1]hept-5-en-2,3-dicarbonsäure und ausgewählten Diaminen gefunden, die sich zu Duroplasten mit ausgezeichneten mechanischen Eigenschaften, wie verbesserter Randfaserdehnung und Biegefestigkeit, verarbeiten lassen. Die gehärteten Produkte zeichnen sich ferner durch eine geringe Wasseraufnahmefähigkeit aus.

Die vorliegende Erfindung betrifft Verbindungen der Formel I 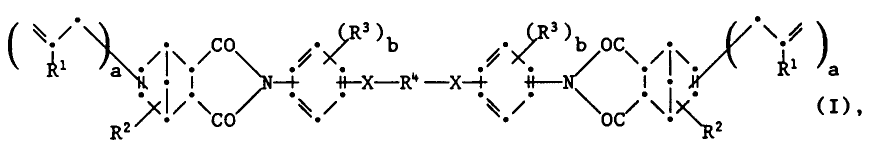
worin a 1, 2 oder 3 ist, b 0, 1 oder 2 bedeutet,
R¹ und R² unabhängig voneinander Wasserstoff oder Methyl sind, R³ C₁-C₆-Alkyl bedeutet, R⁴ eine Gruppe der Formel IIa, IIb, IIc, IId, IIe oder IIf ist 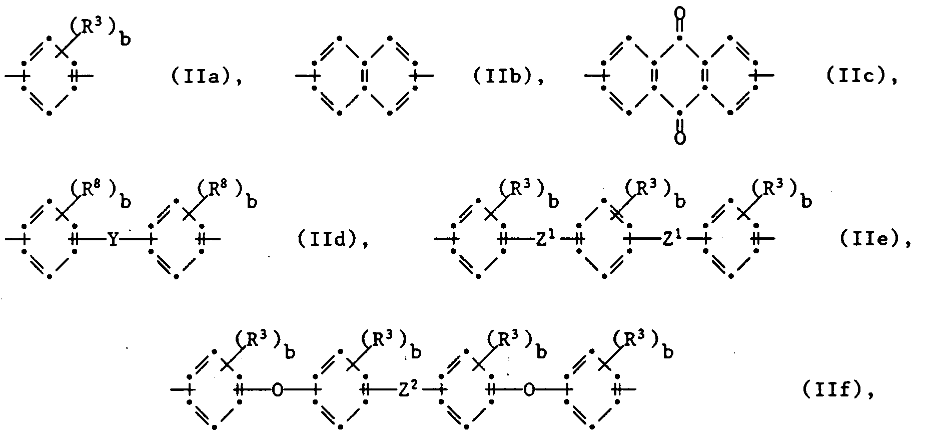
X -O-, -S-, -CO-, -COO-, -CO-NR⁵- oder -CR⁶R⁷- ist, Z¹ -CR⁶R⁷- bedeutet, Z² -SO₂- oder -CO- ist, Y eine direkte C-C-Bindung, -O-, -S-, -SO-, -SO₂-, -CO-, -P(O)R⁹-, -COO-, -CO-NR⁵-, -CₙH₂ₙ- oder -CR¹⁰R¹¹- ist, n eine ganze Zahl von 1 bis 12 ist, R⁵ Wasserstoff oder C₁-C₆-Alkyl ist, R⁶ und R⁷ unabhängig voneinander Wasserstoff, C₁-C₆-Alkyl, -CF₃, Cyclohexyl oder Phenyl bedeuten oder R⁶ und R⁷ zusammen mit dem gemeinsamen C-Atom einen Cycloalkylidenrest mit 5 bis 7 Ringkohlenstoffatomen darstellen, R⁸ C₁-C₆-Alkyl, Chlor oder Brom bedeutet, R⁹ Methyl, Cyclohexyl oder Phenyl ist, R¹⁰ -CF₃, Cyclohexyl oder Phenyl bedeutet, R¹¹ eine der Bedeutungen von R¹⁰ annehmen kann oder zusätzlich Wasserstoff ist oder R¹⁰ und R¹¹ zusammen mit dem gemeinsamen C-Atom einen Cycloalkylidenrest mit 5 bis 7 Ringkohlenstoffatomen darstellen.

Die Indizes a und b und die Reste R¹, R², R³, R⁵ bis R¹¹ können in einem Molekül der Formel I innerhalb der gegebenen Definitionen je unterschiedliche Bedeutungen annehmen.

Bedeuten irgendwelche Reste C₁-C₆-Alkyl, so kann es sich dabei um verzweigte oder vorzugsweise um geradkettige Alkylreste handeln.

Beispiele dafür sind Methyl, Ethyl, n-Propyl, Isopropyl, n-Butyl, sek.-Butyl, tert.-Butyl, n-Pentyl, n-Hexyl oder 1-Ethylbutyl. Bevorzugt wird Methyl.

Beispiele für Cycloalkylidenreste sind Cyclopentyliden, Cycloheptyliden und insbesondere Cyclohexyliden.

Der Index a ist vorzugsweise 1. Der Index b ist vorzugsweise 0.

Der Index n ist vorzugsweise 1 bis 6.

R² ist vorzugsweise Wasserstoff und R³ und R⁸ sind vorzugsweise Methyl.

R⁵ ist vorzugsweise Methyl und insbesondere Wasserstoff.

Der Rest R⁴ ist vorzugsweise eine Gruppe der Formeln IIa, IIb oder IId, wie oben definiert.

X ist bevorzugt -CR⁶R⁷- oder insbesondere -O-.

Das Brückenglied Y ist bevorzugt eine direkte C-C-Bindung, -S-, -SO₂-, -CH₂-, -C(CH₃)₂- oder -CO-, und insbesondere -C(CF₃)₂- und -O-.

Das Brückenglied Z¹ ist vorzugsweise -CH₂- oder insbesondere -C(CH₃)₂-.

Bevorzugte Reste R⁶ und R⁷ sind Wasserstoff, C₁-C₆-Alkyl und -CF₃; ganz besonders bevorzugt sind Wasserstoff, Methyl oder -CF₃.

Bevorzugt sind Verbindungen der Formel I, worin a 1 ist, b 0 ist und R² Wasserstoff bedeutet.

Ferner werden Verbindungen der Formel I bevorzugt, worin R⁴ eine Gruppe der Formel IIa, IIb oder IId ist und b 0 bedeutet.

Besonders bevorzugt werden Verbindungen der Formel I, worin X -C(CH₃)₂- oder insbesondere -O- ist.

In weiteren bevorzugten Verbindungen der Formel I ist Y eine direkte C-C-Bindung, -S-, -SO₂-, -CH₂-, -C(CH₃)₂-, -CO- oder insbesondere -O- oder -C(CF₃)₂- und Z ist -CH₂- oder -C(CH₃)₂-.

Ganz besonders bevorzugt werden Verbindungen der Formel I, worin X -C(CH₃)₂- oder insbesondere -O- ist und R⁴ eine Gruppe der Formeln IIg, IIh oder IIi ist 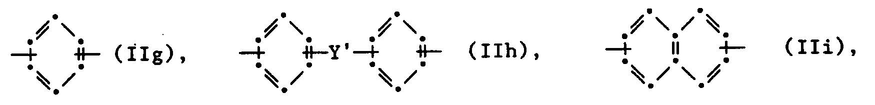
worin Y' eine direkte C-C-Bindung, -O-, -S-, -SO₂-, -C(CF₃)₂- oder -CₙH₂ₙ- ist, n die weiter oben definierte Bedeutung besitzt, die freien Valenzen im Rest IIg in 1,2- oder 1,4-Position zueinander stehen, im Rest IIh in 1,2- oder 1,4-Position zur jeweiligen Brücke stehen und im Rest IIi in 1,5-, 2,6- oder 2,7-Position zueinander stehen.

In weiteren ganz besonders bevorzugten Verbindungen der Formel I besitzt X die Bedeutung -O- und R⁴ ist ein Rest der Formel IIj 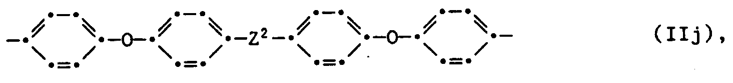
worin Z² die oben definierte Bedeutung besitzt.

Besonders bevorzugt werden Verbindungen der Formel III 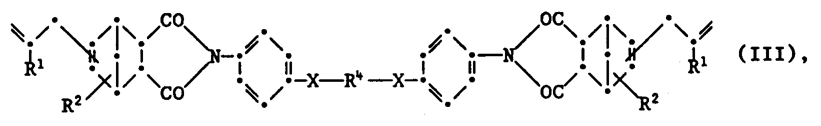
worin R¹, R², R⁴ und X die oben definierte Bedeutung besitzen.

Ganz besonders bevorzugt wird die Verbindung der Formel IIIa 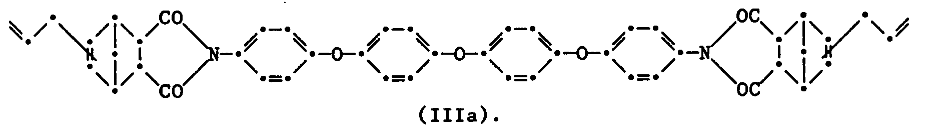
Die erfindungsgemässen Bisimide können in Analogie zu bekannten Verfahren durch Umsetzung eines Anhydrids der Formel IVa 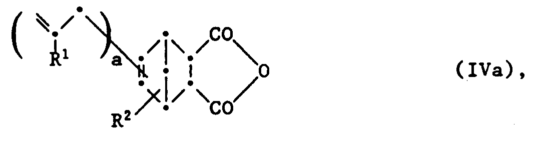
bei erhöhter Temperatur unter Abdestillieren des bei der Reaktion entstehenden Wassers mit einem Diamin der Formel V 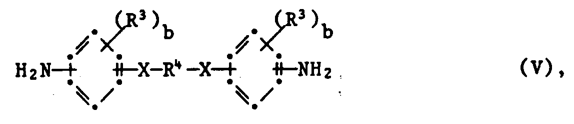
worin R¹, R², R³, R⁴, X, a und b die weiter oben definierte Bedeutung besitzen, dargestellt werden.

Die Diamine der Formel V werden zweckmässigerweise in stöchiometrischem Verhältnis eingesetzt. Die Umsetzung kann ohne Lösungsmittel oder vorzugsweise in Gegenwart eines inerten organischen Lösungsmittels, das für die azeoptrope Entfernung des Wassers verwendbar ist (Schleppmittel) erfolgen. Als Lösungsmittel eignen sich beispielsweise aromatische Kohlenwasserstoffe, wie Toluol, Xylol oder Mesitylen, oder halogenierte aromatische Kohlenwasserstoffe, wie Chlorbenzol oder Dichlorbenzol.

Die Umsetzung wird in der Regel bei Temperaturen zwischen 110 und 200°C durchgeführt. Die Reaktionsdauer ist von der Geschwindigkeit der Wasserabspaltung abhängig und beträgt in der Regel etwa 2 bis 20 Stunden.

Die Anhydride der Formel IV können gemäss dem in der US-A-3,105,839 beschriebenen Verfahren hergestellt werden, indem man Natriumcyclopentadienid oder Natriummethylcyclopentadienid mit einem Allyl- oder Methallylhalogenid, vorzugsweise einem -chlorid, umsetzt, worauf sich eine Diels-Alder Reaktion mit Maleinsäureanhydrid anschliesst.

Anhydride der Formel IV mit mehreren Allyl- oder Methallylgruppen können in Analogie zum obigen Verfahren durch Umsetzung mit einem stöchiometrischen Ueberschuss Allyl- oder Methallylhalogenid erhalten werden. Obgleich in der US-A-3,105,839 angegben wird, dass die (Meth)allylgruppe in 7-Stellung des bicyclischen Systems gebunden ist, zeigen neuere Untersuchungen, dass eine isomere Mischung in Bezug auf die Stellung der Allyl- oder Methallylgruppe und auch auf die Endo- und Exokonfiguration des Anhydridteils gebildet wird.

Einige der erfindungsgemässen Bisimide, worin X -O- ist, R⁴ ein Rest der Formel IId ist und Y -CO- oder -SO₂- ist, können auch durch Umsetzung von im wesentlichen zwei Aequivalenten eines Phenols der Formel IVb mit einem Dibromid, Dichlorid oder Difluorid der Formel IVc in Gegenwart einer Base, wie K₂CO₃, bei erhöhter Temperatur hergestellt werden 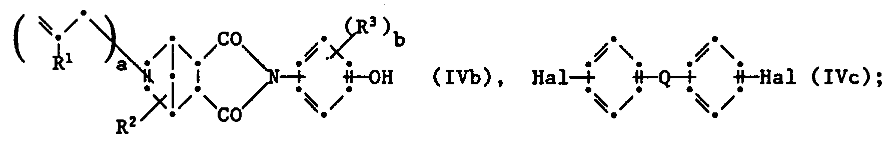
dabei besitzen R¹, R², R³, a und b die weiter oben definierte Bedeutung, Hal ist F, Cl oder Br und Q ist -CO- oder -SO₂-.

Die Umsetzung erfolgt zweckmässig in einem inerten organischen Lösungsmittel bei erhöhten Temperaturen. Beispiele dafür sind weiter oben angegeben.

Die Verbindungen der Formel IVb sind beispielsweise aus der EP-A-166,693 bekannt. Die Verbindungen der Formel IVc sind ebenfalls an sich bekannt und teilweise käuflich erhältlich.

Die Diamine der Formel V sind teilweise bekannt oder lassen sich in Analogie zu den bekannten Diaminen herstellen.

So lassen sich die Verbindungen der Formel Va, 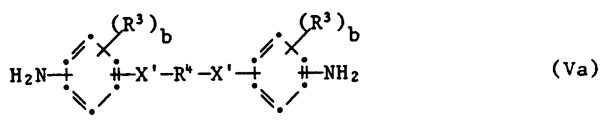
worin X' -S- oder -O- ist und R³, R⁴ und b die oben definierte Bedeutung besitzen, beispielsweise herstellen, indem man ein Bisphenol HX'-R⁴-X'H in Gegenwart einer Base, beispielsweise K₂CO₃, mit einem Chlor- oder Bromnitrobenzol umsetzt, und die Dinitroverbindung mit Wasserstoff in Gegenwart eines an sich bekannten Hydrierungskatalysators, wie Pd/Kohle, Raney-Nickel oder Fe/HCl, zum entsprechenden Diamin hydriert.

Die Verbindungen der Formel Va, worin X' -COO- ist, lassen sich beispielsweise durch Veresterung einer Dicarbonsäure HOOC-R⁴ -COOH oder einer ihrer esterbildenden Derivate, wie des Säurechlorids, mit einem Aminophenol direkt erhalten; oder die Herstellung erfolgt durch Umsetzung eines Nitrophenols mit einer Dicarbonsäure HOOC-R⁴-COOH oder einem ihrer esterbildenden Derivate und anschliessende Hydrierung zum Diamin.

Die Verbindungen der Formel Va, worin X' -COO- ist, lassen sich sich auch durch Umsetzung eines Nitrobenzoesäurechlorids mit einem Bisphenol HO-R⁴-OH und anschliessender Reduktion zum Diamin herstellen.

Die Verbindungen der Formel Va, worin X' -CO-NR⁵- ist, lassen sich beispielsweise durch Umsetzung eines Diamins HR⁵N-R⁴-NR⁵H mit einem Nitrobenzoesäurechlorid und anschliessende Hydrierung der Dinitroverbindung zum Diamin erhalten. Beispiele für solche Umsetzungen findet man in J. Polymer Sci.; Part A-1, 4(9), 2093-2105 (1966) oder der GB-A-979,407.

Die Verbindungen der Formel Va, worin X' -CO-NR⁵- ist, lassen sich auch erhalten, indem man eine Dicarbonsäure HOOC-R⁴-COOH oder eines ihrer amidbildenden Derivate, wie des Esters oder des Säurechlorids, mit einem Nitroanilin umsetzt und anschliessend die Dinitroverbindung zum Diamin reduziert. Beispiele für solche Umsetzungen sind in der GB-A-1,227,387 zu finden.

Die Verbindungen der Formel Va, worin X' -CO- ist, lassen sich beispielsweise durch Umsetzung eines Nitrobenzoylchlorids mit einer aromatischen Verbindung H-R⁴-H in Gegenwart von AlCl₃ und anschliessender Hydrierung der Dinitroverbindung zum Diamin erhalten.

Die Verbindungen der Formel Va, worin X' -CR⁶R⁷- ist, lassen sich durch Kondensation einer aromatischen Verbindung H-R⁴-H mit einem Aldehyd oder Keton R⁶-CO-R⁷ und einem Aminobenzol in alkalischem Medium in an sich bekannter Weise erhalten.

Diese Umsetzungen werden zweckmässigerweise in Gegenwart eines inerten organischen Lösungsmittels durchgeführt. Beispiele dafür sind aprotisch polare Lösungsmittel, wie Dimethylformamid (DMF), Dimethylacetamid (DMA), N-Methylpyrrolidon (NMP), Dimethylsulfoxid (DMSO) oder cyclische Harnstoffe, wie Tetramethylenharnstoff.

Die Umsetzungen werden, je nach Reaktivität der eingesetzten Ausgangsprodukte im allgemeinen bei 100 bis 200°C vorgenommen. Die Reaktionsdauer beträgt in der Regel 2 bis 20 Stunden.

Die Ausgangsverbindungen zur Herstellung der Diamine der Formel V sind an sich bekannt.

Die Diamine der Formel V sind teilweise bekannt, beispielsweise aus der US-A-4,517,321 oder aus der US-A-4,196,144.

Die neuen Diamine der Formeln VIa, VIb, VIc und VId sind ebenfalls ein Gegenstand der vorliegenden Erfindung 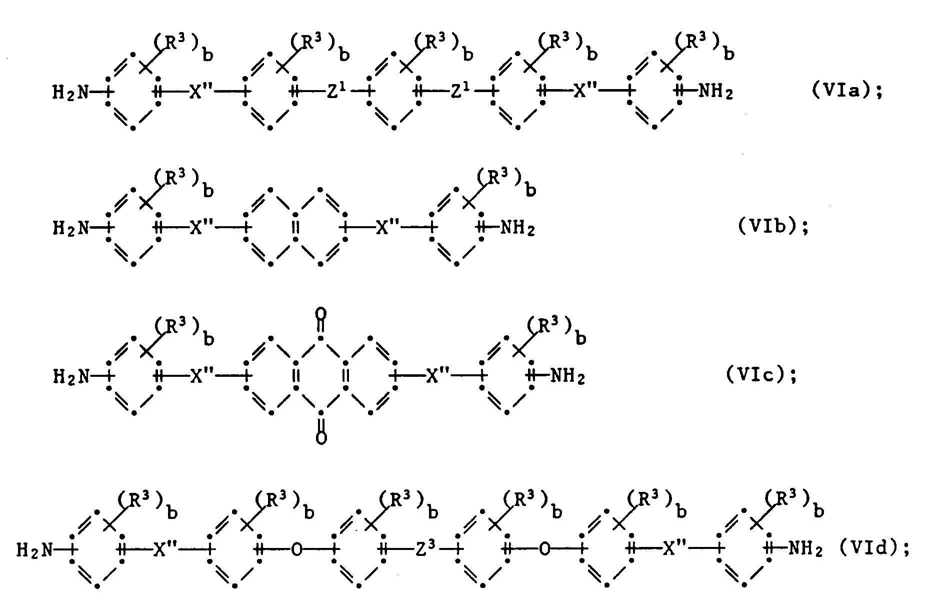
dabei besitzen R³, Z¹ und b die weiter oben definierte Bedeutung, X'' ist -O-, -S-, -CO- oder -COO- und Z³ ist -CO-.

Ferner betrifft die Erfindung die Dinitrozwischenprodukte der Formeln VIIa, VIIb, VIIc und VIId, welche in der oben beschriebenen Weise hergestellt werden können und als Ausgangsprodukte zur Herstellung ausgewählter Diamine der Formeln VIa, VIb, VIc und VId eingesetzt werden können 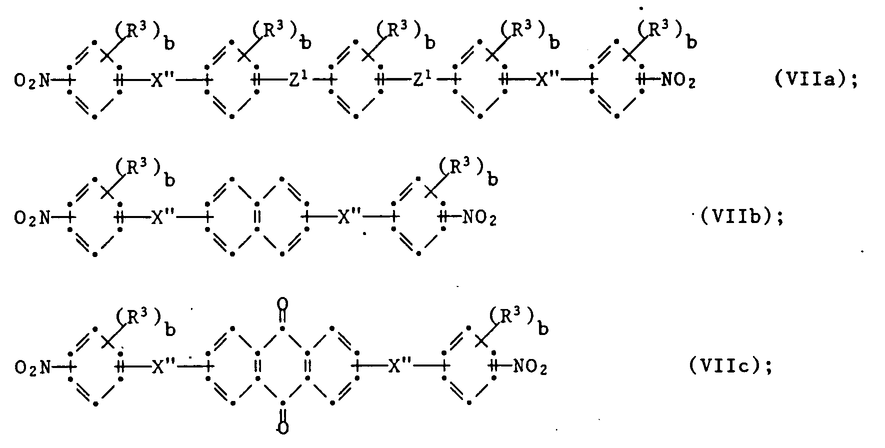 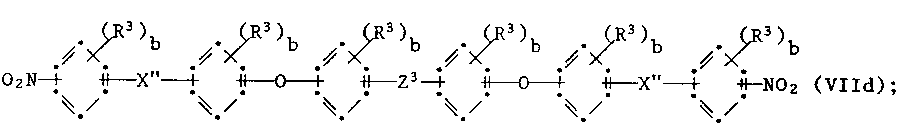
dabei besitzen R³, Z¹, b und X'' die weiter oben definierte Bedeutung und Z³ is -CO-.

Die Verbindungen der Formel I sind flüssige oder niedrigschmelzende feste Stoffe, die zu festen Produkten mit hoher Glasumwandlungstemperatur und Wärme- und Wasserbeständigkeit polymerisiert werden können.

Diese Produkte können vielseitig angewendet werden, beispielsweise als Laminier- oder Elektroharze, als Hochtemperaturklebstoffe oder zur Herstellung von Beschichtungen oder Formkörpern, wie Prepregs oder Verbundwerkstoffen.

Gegenstand der Erfindung ist somit auch die Verwendung der erfindungsgemässen Verbindungen der Formel I zur Herstellung von Formkörpern, Beschichtungen oder Verklebungen.

Die erfindungsgemässen Verbindungen können direkt verwendet und polymerisiert werden,
oder sie können zunächst in einem inerten, organischen Lösungsmittel, wie DMF, DMA, NMP, Toluol, Xylol, Methylethylketon, Ethylenglykolmonoalkyl- und -dialkylethern mit 1-4 C-Atomen in den Alkylgruppen oder einem ähnlichen, in der Lackindustrie üblichen Lösungsmittel, gelöst werden.

Solche Lösungen können als Imprägniermittel oder als Beschichtungsmittel oder auch als Versandobjekt an den Verbraucher dienen.

Die Verbindungen der Formel I können zu neuen Polymeren umgesetzt werden, die sich durch die oben erwähntenvorteilhaften Eigenschaften auszeichnen.

Gegenstand der Erfindung sind also auch die gehärteten Produkte erhältlich durch Erhitzen der Verbindungen der Formel I.

Zur Polymerisation erhitzt man die Verbindungen der Formel I vorteilhafterweise auf Temperaturen zwischen 180 und 300°C, insbesondere zwischen 200 und 250°C. Die Dauer der Polymerisation liegt je nach Temperatur zweckmässigerweise zwischen 6 und 60 Stunden.

Den Verbindungen der Formel I können auch Härtungskatalysatoren zugegeben werden,
beispielsweise organische Peroxide, wie Di-tert.-butylperoxid, Dicumylperoxid oder tert.Butylperbenzoat; oder Lewis-Säuren, wie FeCl₃, ZnCl₂, BCl₃, BF₃, AlCl₃, TiCl₄, SnCl₄ oder SbCl₅.

Selbstverständlich können den Bisimiden der Formel I vor der Polyinerisation inerte
und stabile Zusatzstoffe, wie Füllmittel, Verstärkungsmittel, insbesondere Glasfasern, Kohlenstoffasern oder Aramidfasern, Weichmacher, Pigmente, Farbstoffe, Formtrennmittel, flammhemmende Stoffe und andere übliche Zusatzstoffe zugegeben werden.

Die folgenden Beispiele erläutern die Erfindung:

### A. Herstellung der Bisimide der Formel I

### Beispiel A1: 2,2-Bis-[4-(4-allyl-bicyclo[2.2.1]hept-5-en-2,3-dicarbonsaureimidylphenoxy)-phenyl]-hexafluoropropan

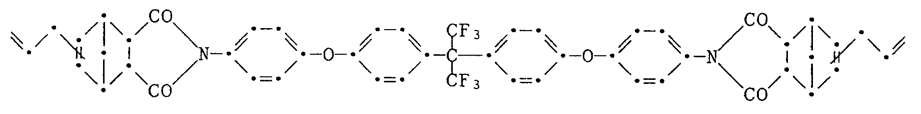

In einem 6 l Sulfierkolben, ausgerüstet mit Thermometer, Flügelrührer, Azeotropaufsatz und Intensivkühler, werden 259,2 g (0,5 Mol) 2,2-Bis-[4-(4-aminophenoxy)-phenyl]-hexafluoropropan, 204,2 g (1,0 Mol) Allylbicyclo[2.2.1]hept-5-en-2,3-dicarbonsäureanhydrid, hergestellt gemäss US-A-3,105,839, in 1200 ml Xylol 6 Stunden und 40 Minuten lang bei 90 bis 136°C zur Reaktion gebracht und das entstehende Reaktionswasser azeotrop ausgekreist. Nach beendeter Reaktion wird die Lösung bei 80 bis 95°C am Wasserstrahlvakuum eingeengt und anschliessend bei 120 bis 160°C/0,2 mbar bis zur Gewichtskonstanz getrocknet.
Man erhält 433,1 g (97,2 % d.Th.) eines glasigen, rotbraunen Bis-Allylnadicimids mit folgenden analytischen Daten:

| Elementaranalyse: | | | | |
|---|---|---|---|---|
| | %C | %H | %N | %F |
| berechnet: | 68,76 | 4,53 | 3,14 | 12,80 |
| gefunden: | 68,57 | 4,66 | 3,32 | 12,90. |

Molekulargewicht (dampfdruckosmometrisch): 891.
Viskosität (bei 150°C): 5840 mPas.

### Beispiel A2: 4,4'-Bis-(4-allyl-bicyclo[2.2.1]hept-5-en-2,3-dicarbonsäureimidylphenoxy)-diphenylether

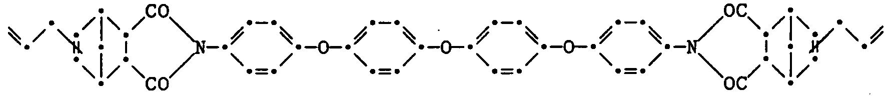

Analog Beispiel A1 werden 576,7 g (1,5 Mol) 4,4'-Bis-(4'-aminophenoxy)-diphenylether, 612,7 g (3,0 Mol) Allyl-bicyclo[2.2.1]hept-5-en-2,3-dicarbonsäureanhydrid in 3000 ml Xylol 9 Stunden und 30 Minuten lang zur Reaktion gebracht und anschliessend gemäss Beispiel A1 aufgearbeitet. Es werden 1106 g (97,4 % d.Th.) eines braunen, glasigen Produktes erhalten, dessen Viskosität (bei 150°C) 2560 mPas beträgt.

| Elementaranalyse: | | | |
|---|---|---|---|
| | %C | %H | %N |
| berechnet: | 76,17 | 5,33 | 3,70 |
| gefunden: | 75,69 | 5,40 | 3,50. |

Wasserstoffaufnahme:
5,29 mMol H₂/g (berechnet), 4,6 mMol H₂/g (gefunden).
Molekulargewicht (GPC): Mₙ = 779; M_{w} = 913.

### Beispiel A3: 4,4'-Bis-(4-allyl-bicyclo[2.2.1]hept-5-en-2,3-dicarbonsäureimidylphenoxy)-diphenylsulfid

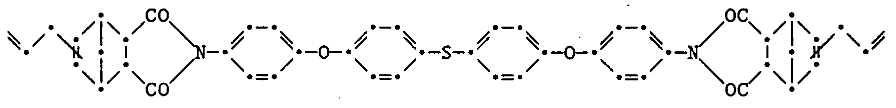

200,25 g (0,5 Mol) 4,4'-Bis-(4-aminophenoxy)-diphenylsulfid und 204,2 g (1,0 Mol) Allyl-bicyclo[2.2.1]hept-5-en-2,3-dicarbonsäureanhydrid werden in 1150 ml Xylol bei 130 - 135°C 6 Stunden und 20 Minuten lang umgesetzt und wie in Beispiel A1 beschrieben aufgearbeitet. Man erhält 386,1 g (99,9 % d.Th.) des gewünschten Produktes mit folgenden analytischen Daten:

| Elementaranalyse: | | | | |
|---|---|---|---|---|
| | %C | %H | %N | %S |
| berechnet: | 74,59 | 5,22 | 3,62 | 4,15 |
| gefunden: | 74,35 | 5,28 | 3,44 | 4,26. |

Molekulargewicht (GPC): Mₙ = 794; M_{w} = 842.
Viskosität (bei 150°C): 940 mPas.

### Beispiel A4: 4,4'-Bis-(4-allyl-bicyclo[2.2.1]hept-5-en-2,3-dicarbonsäureimidylphenoxy)-diphenylmethan

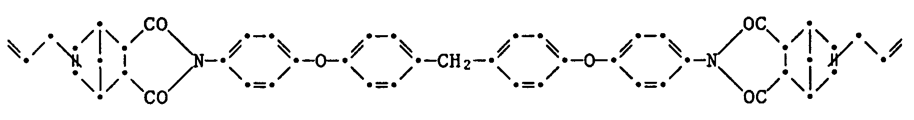

Wie in Beispiel A1 beschrieben werden 95,6 g (0,25 Mol) 4,4'-Bis-(4-aminophenoxy)-diphenylmethan und 102.1 g (0,5 Mol) Allyl-bicyclo[2.2.1]-hept-5-en-2,3-dicarbonsäureanhydrid in 600 ml Xylol 4 Stunden und 5 Minuten lang bei 128 - 136°C zur Reaktion gebracht und das Produkt analog Beispiel A1 isoliert. Man erhält 186,9 (99 % d.Th.) eines bräunlichen, glasigen Bis-allylnadicimids.

| Elementaranalyse: | | | |
|---|---|---|---|
| | %C | %H | %N |
| berechnet: | 77,96 | 5,61 | 3,71 |
| gefunden: | 77,44 | 5,79 | 3,55. |

Wasserstoffaufnahme:
5,30 mMol H₂/g (berechnet), 4,70 mMol H₂/g (gefunden).
Viskosität (bei 150°C): 920 mPas.

### Beispiel A5: 1,3-Bis-(4-allyl-bicyclo[2.2.1]hept-5-en-2,3-dicarbonsäureimidylphenoxy)-benzol

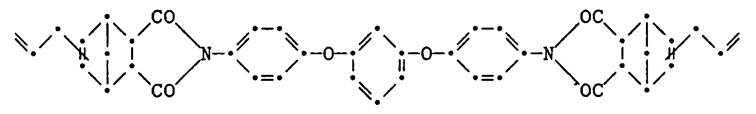

435 g (1,488 Mol) 1,3-Bis-(4-aminophenoxy)-benzol und 607,7 g (2,976 Mol) Allyl-bicyclo[2.2.1]hept-5-en-2,3-dicarbonsäureanhydrid in 3420 ml Xylol setzt man gemäss Beispiel A1 um und arbeitet analog auf. Es werden 988,6 g (99,9 % d.Th.) eines bräunlichen, glasigen Produktes erhalten, dessen Viskosität (bei 150°C) 700 mPas beträgt.

| Elementaranalyse: | | | |
|---|---|---|---|
| | %C | %H | %N |
| berechnet: | 75,89 | 5,46 | 4,21 |
| gefunden: | 75,29 | 5,49 | 4,12. |

Wasserstoffaufnahme:
6,02 mMol H₂/g (berechnet), 5,2 mMol H₂/g (gefunden).
Molekulargewicht (dampfdruckosmometrisch): 866.

### Beispiel A6: 1,4-Bis-(4-allyl-bicyclo[2.2.1]hept-5-en-2,3-di-carbonsäureimidylphenoxy)-benzol

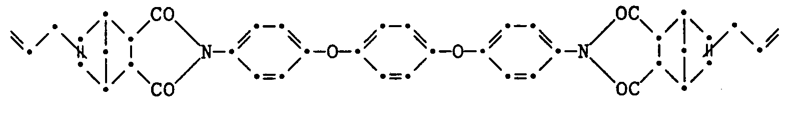

Wie in Beispiel A1 beschrieben werden 146,1 g (0,5 Mol) 1,4-Bis-(4-aminophenoxy)-benzol und 204,2 g (1,0 Mol) Allyl-bicyclo[2.2.1]hept-5-en-2,3-dicarbonsäureanhydrid in 1150 ml Xylol umgesetzt und aufgearbeitet. Man erhält 332 g (99,9 % d.Th.) des gewünschten Bis-allylnadicimids mit einer Viskosität (bei 150°C) von 1080 mPas.

| Elementaranalyse: | | | |
|---|---|---|---|
| | %C | %H | %N |
| berechnet: | 75,89 | 5,46 | 4,21 |
| gefunden: | 75,79 | 5,55 | 4,10. |

Wasserstoffaufnahme:
6,02 mMol H₂/g (berechnet), 5,3 mMol H₂/g (gefunden).

### Beispiel A7: 1,3-Bis-[1-(4-allyl-bicyclo[2.2.1]hept-5-en-2,3-dicarbonsäureimidylphenyl)-1-methylethyliden]-benzol

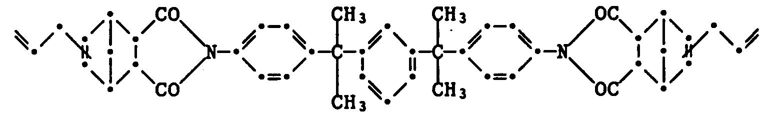

68,9 g (0,2 Mol) eines technisch hergestellten 1,3-Bis-[1-(4-aminophenyl)-1-methylethyliden]-benzols und 81,7 g (0,4 Mol) Allyl-bicyclo-[2.2.1]hept-5-en-2,3-dicarbonsäureanhydrid in 450 ml Xylol werden analog Beispiel A1 zur Reaktion gebracht und isoliert. Es wird ein bräunliches, glasiges Bisimid erhalten, dessen Erweichungspunkt 86°C beträgt.

| Elementaranalyse: | | | |
|---|---|---|---|
| | %C | %H | %N |
| berechnet: | 80,42 | 6,75 | 3,91 |
| gefunden: | 79,95 | 6,83 | 3,62. |

Wasserstoffaufnahme:
5,58 mMol H₂/g (berechnet), 5,1 mMol H₂/g (gefunden).

### Beispiel A8: 1,4-Bis-{1-[4-(4-allyl-bicyclo[2.2.1]hept-5-en-2,3-dicarbonsäureimidylphenoxy)-phenyl]-1-methylethyliden}-benzol

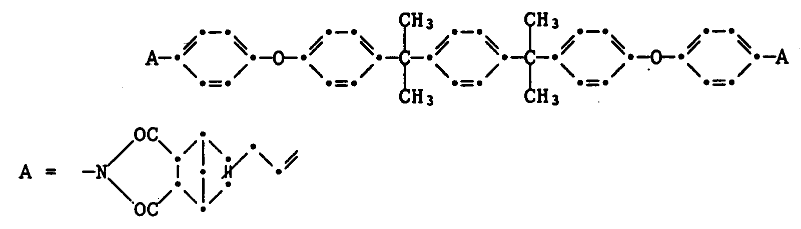

Gemäss Beispiel A1 werden 292,2 g (0,5 Mol) 1,4-Bis-{1-[4-(4-aminophenoxy)-phenyl]-1-methylethyliden}-benzol und 204,2 g (1,0 Mol) Allylbicyclo[2.2.1]hept-5-en-2,3-dicarbonsäureanhydrid in 1200 ml Xylol umgesetzt und aufgearbeitet. Es werden 448,9 g (99,6 % d.Th.) des gewünschten Bisimids erhalten, dessen Viskosität (bei 150°C) 5440 mPas beträgt.

| Elementaranalyse: | | | |
|---|---|---|---|
| | %C | %H | %N |
| berechnet: | 79,97 | 6,26 | 3,11 |
| gefunden: | 79,55 | 6,22 | 3,07. |

Wasserstoffaufnahme:
4,44 mMol H₂/g (berechnet), 4,0 mMol H₂/g (gefunden).

### Beispiel A9: 2,2'-Bis-(4-allyl-bicyclo[2.2.1]hept-5-en-2,3-dicarbonsäureimidylphenoxy)-biphenyl

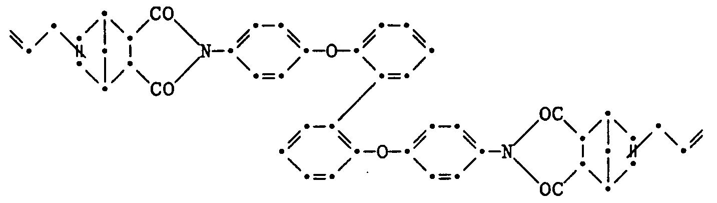

408 g (2 Mol) Allyl-bicyclo[2.2.1]hept-5-en-2,3-di-carbonsäureanhydrid und 368 g (1 Mol) 2,2'-Bis-(4-aminophenoxy)-biphenyl werden in 2000 ml Toluol vorgelegt und 16 Stunden am Wasserabscheider unter Rückfluss erhitzt. Anschliessend wird wie in Beispiel A1 beschrieben aufgearbeitet. Man erhält 728 g (98 % d.Th.) eines rotbraunen, bei Raumtemperatur festen Harzes mit einem Erweichungspunkt von 78°C.

| Elementaranalyse: | | | |
|---|---|---|---|
| | %C | %H | %N |
| berechnet: | 77,82 | 5,44 | 3,78 |
| gefunden: | 77,58 | 5,57 | 3,65. |

Molekulargewicht (GPC): Mₙ = 563; M_{w} = 576.

### Beispiel A10: 2,2-Bis-{4-(4-allyl-bicyclo[2.2.1]hept-5-en-2,3-dicarbonsäureimidylphenoxy)-phenyl}-propan

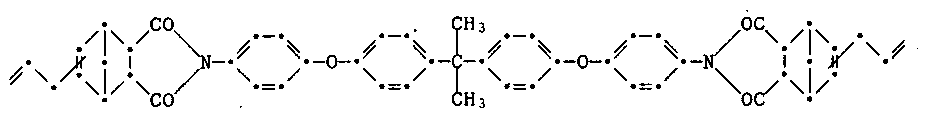

61,5 g (0,15 Mol) 2,2-Bis-{4-(4-aminophenoxy)-phenyl}-propan und 61,2 g (0,3 Mol) Allyl-bicyclo[2.2.1]hept-5-en-2,3-dicarbonsäureanhydrid werden in 400 ml Xylol gemäss Beispiel A1 bei 109-131°C 5 Stunden und 35 Minuten lang zur Reaktion gebracht und analog aufgearbeitet. Es werden 116,2 g (99 % der Theorie) eines braunen, glasigen Harzes erhalten, dessen Viskosität (bei 180°C) 430 mPas beträgt.

| Elementaranalyse: | | | |
|---|---|---|---|
| | %C | %H | %N |
| berechnet: | 78,24 | 5,92 | 3,58 |
| gefunden: | 78,00 | 6,07 | 3,44. |

Molekulargewicht (GPC): Mₙ = 779; M_{w} = 796.
- Wasserstoffaufnahme:: 5,11 mMol H2/g (berechnet);
4,8 mMol H2/g (gefunden).

### Beispiel A11: 4,4'-Bis-(4-allyl-bicyclo[2.2.1]hept-5-en-2,3-dicarbonsäureimidylphenoxy)-biphenyl

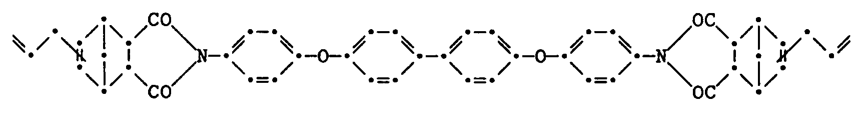

Analog Beispiel A1 werden 110,5 g (0,3 Mol) 4,4'-Bis-(4-aminophenoxy)-biphenyl und 122,5 g (0,6 Mol) Allyl-bicyclo[2.2.1]hept-5-en-2,3-dicarbonsäureanhydrid in 200 ml Xylol 17 Stunden lang bei 129-136°C umgesetzt und wie in Beispiel A1 beschrieben aufgearbeitet. Man erhält 219,1 g (98,6 % der Theorie) eines braunen, festen Bisimids, dessen Viskosität (bei 180°C) 1140 mPas beträgt.

| Elementaranalyse: | | | |
|---|---|---|---|
| | %C | %H | %N |
| berechnet: | 77,82 | 5,44 | 3,78 |
| gefunden | 77,07 | 5,49 | 3,63. |

### Beispiel A12: 4,4'-Bis-(4-allyl-bicyclo[2.2.1]hept-5-en-2,3-dicarbonsäureimidylphenoxy)-diphenylsulfon

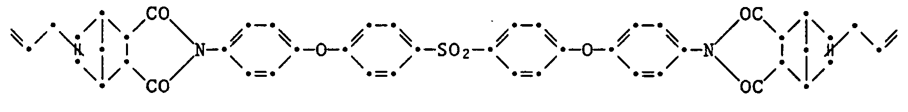

129,8 g (0,3 Mol) 4,4'-Bis-(4-aminophenoxy)-diphenylsulfon und 122,5 g (0,6 Mol) Allyl-bicyclo[2.2.1]hept-5-en-2,3-dicarbonsäureanhydrid werden in 200 ml Xylol 18 Stunden und 40 Minuten lang bei 119-137°C reagieren gelassen und analog Beispiel A1 aufgearbeitet. Man erhält 237,2 g (98,2 % der Theorie) des gewünschten Bisimids, dessen Viskosität (bei 180°C) 5860 mPas beträgt.

| Elementaranalyse: | | | | |
|---|---|---|---|---|
| | %C | %H | %N | %S |
| berechnet: | 71,63 | 5,01 | 3,48 | 3,98 |
| gefunden: | 70,67 | 5,11 | 3,61 | 3,90. |

Molekulargewicht (GPC): Mₙ = 832; M_{w} = 898.

### Beispiel A13: 4,4'-Bis-((4-allyl-bicyclo[2.2.1]hept-5-en-2,3-dicarbonsäureimidylphenoxy)-phenoxy)-diphenylsulfon

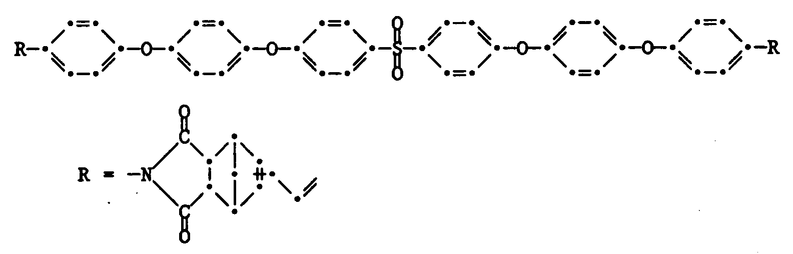

8,0 g (0,0129 Mol) Bis-(4-(4-aminophenoxy)-phenoxy)-diphenylsulfon und 5,27 g (0,0258 Mol) Allyl-bicyclo[2.2.1]hept-5-en-2,3-dicarbonsäureanhydrid werden in 30 ml Xylol und 30 ml Dimethylacetamid gemäss Beispiel A1 bei 143°C-152°C 19 Stunden und 40 Minuten lang umgesetzt und das entstehende Reaktionswasser azeotrop ausgekreist.

Analog Beispiel A1 wird aufgearbeitet und 11,3 g (88,3 % der Theorie) eines glasigen, festen Imids erhalten, dessen Viskosität bei 180°C 2440 mPas beträgt.

| Elementaranalyse: | | | |
|---|---|---|---|
| | %C | %H | %N |
| berechnet: | 72,86 | 4,89 | 2,83 |
| gefunden: | 72,45 | 5,23 | 2,95. |

¹ Gemessen mittels Thermoanalyser DuPont 9900

### B. Anwendungsbeispiele

### Beispiel B1: Ein gemäss Synthesebeispiel A2 hergestelltes Bis-allylnadicimid wird bei 200°C im Vakuum entgast und in eine auf 200°C vorgewärmte Form von 120 x 120 x 4 mm gegossen. Die Formkörper werden 3 Stunden bei 200°C, 3 Stunden bei 220°C und 6 Stunden bei 250°C gehärtet. Nach dem Abkühlen werden die Platten in Prüfstäbe zerschnitten und folgende Eigenschaften gemessen: Biegefestigkeit (nach ISO 178): 132 N/mm². Randfaserdehnung (nach ISO 178): 6,4 %. Zugscherfestigkeit (nach ISO 4587): 10,5 N/mm². Glasumwandlungstemperatur (TMA)¹: 238°C.

### Beispiel B2: Das gemäss Beispiel A6 hergestellte Bisimid giesst man als heisses, dünnflüssiges Harz in ein Reagenzglas und härtet 3 Stunden bei 200°C, 3 Stunden bei 220°C und 6 Stunden bei 250°C. Man erhält einen klaren Festkörper mit einer Glasumwandlungstemperatur von 251°C.

### C. Herstellung der Zwischenprodukte der Formeln VI und VII

### Beispiel C1: 1,4-Bis-{1-[4-(4-nitrophenoxy)-phenyl]-1-methyl-ethyliden}-benzol

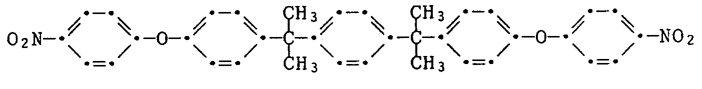

692,9 g (2,0 Mol) Bisphenol-P der Firma Mitsui Petrochemical Industries Ltd., 630,3 g (4,0 Mol) 1-Chlor-4-nitrobenzol, 608,2 g (4,4 Mol) wasserfreies Kaliumcarbonat werden in 2000 ml Dimethylformamid 5 Stunden und 10 Minuten lang bei 138 bis 139°C umgesetzt. Nach beendeter Reaktion wird die noch heisse Reaktionslösung unter Rühren in 15 Liter Wasser eingetragen, das augefallene Produkt abgesaugt, mit Wasser gewaschen und der Rückstand bei 110°C im Vakuum getrocknet.
Man erhält 1142,3 g (97 % d.Th.) einer gelblichen, kristallinen Dinitroverbindung, die bei 195°C schmilzt.

| Elementaranalyse: | | | |
|---|---|---|---|
| | %C | %H | %N |
| berechnet: | 73,45 | 5,48 | 4,76 |
| gefunden: | 72,93 | 5,57 | 4,72. |

### Beispiel C2: 1,4-Bis-{1-[4-(4-aminophenoxy)phenyl]-1-methyl-ethyliden}-benzol

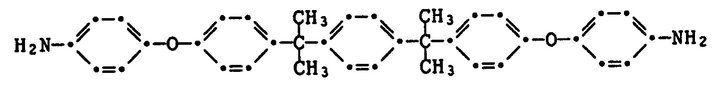

In einer Suspension von 33 g 5%iger Pd-Kohle in 11 Liter Dimethylformamid werden 1190 g (2,02 Mol) der im Beispiel C1 beschriebenen Dinitroverbindung 1,5 Stunden lang bei 45°C in Gegenwart von Wasserstoff (4 bar) reduziert. Anschliessend wird die Reaktionslösung filtriert und das Filtrat in 25 Litern Wasser ausgefällt. Man extrahiert das Produkt 3 mal mit Chloroform, vereinigt die organischen Phasen, trocknet die Lösung mit wasserfreiem Natriumsulfat, filtriert und engt das Filtrat am Rotationsverdampfer bei 70°C/Vakuum ein. Der Rückstand wird bei 120°C/0,2 mbar bis zur Gewichtskonstanz getrocknet und man erhält 907,3 g (92,7 % d.Th.) des gewünschten Diamins, das bei 190°C schmilzt.

| Elementaranalyse: | | | |
|---|---|---|---|
| | %C | %H | %N |
| berechnet: | 81,79 | 6,86 | 5,30 |
| gefunden: | 81,71 | 6;72 | 5,25. |

## Patentansprüche

1. Verbindungen der Formel I 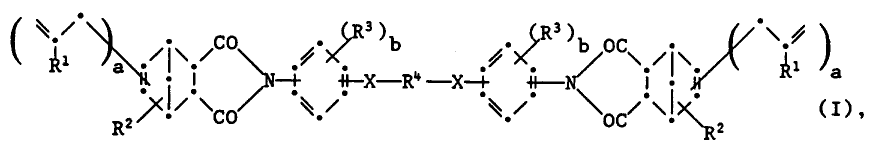 worin a 1, 2 oder 3 ist, b 0, 1 oder 2 bedeutet, R¹ und R² unabhängig voneinander Wasserstoff oder Methyl sind, R³ C₁-C₆-Alkyl bedeutet, R⁴ eine Gruppe der Formel IIa, IIb, IIc, IId, IIe oder IIf ist 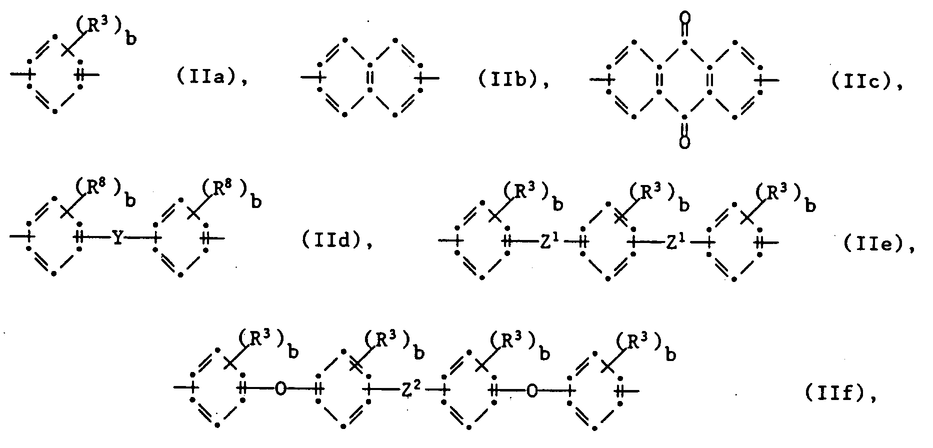 X -O-, -S-, -CO-, -COO-, -CO-NR⁵- oder -CR⁶R⁷- ist, Z¹ -CR⁶R⁷- bedeutet, Z² -SO₂- oder -CO- ist, Y eine direkte C-C-Bindung, -O-, -S-, -SO-, -SO₂-; -CO-, -P(O)R⁹-, -COO-, -CO-NR⁵-, -CₙH₂ₙ- oder -CR¹⁰R¹¹- ist, n eine ganze Zahl von 1 bis 12 ist, R⁵ Wasserstoff oder C₁-C₆-Alkyl ist, R⁶ und R⁷ unabhängig voneinander Wasserstoff, C₁-C₆-Alkyl, -CF₃, Cyclohexyl oder Phenyl bedeuten oder R⁶ und R⁷ zusammen mit dem gemeinsamen C-Atom einen Cycloalkylidenrest mit 5 bis 7 Ringkohlenstoffatomen darstellen, R⁸ C₁-C₆-Alkyl, Chlor oder Brom bedeutet, R⁹ Methyl, Cyclohexyl oder Phenyl ist, R¹⁰ -CF₃, Cyclohexyl oder Phenyl bedeutet, R¹¹ eine der Bedeutungen von R¹⁰ annehmen kann oder zusätzlich Wasserstoff ist oder R¹⁰ und R¹¹ zusammen mit dem gemeinsamen C-Atom einen Cycloalkylidenrest mit 5 bis 7 Ringkohlenstoffatomen darstellen.

2. Verbindungen der Formel I gemäss Anspruch 1, worin a 1 ist, b 0 ist und R² Wasserstoff bedeutet.

3. Verbindungen der Formel I gemäss Anspruch 1, worin R⁴ eine Gruppe der Formel IIa, IIb oder IId ist und b 0 bedeutet.

4. Verbindungen der Formel I gemäss Anspruch 1, worin X -C(CH₃)₂- oder insbesondere -O- ist.

5. Verbindungen der Formel I gemäss Anspruch 1, worin Y eine direkte C-C-Bindung, -S-, -SO₂-, -CH₂-, -C(CH₃)₂-, -CO- oder insbesondere -O-oder -C(CF₃)₂- ist und worin Z -CH₂- oder -C(CH₃)₂- ist.

6. Verbindungen der Formel I gemäss Anspruch 1, worin X -C(CH₃)₂- oder insbesondere -O- ist und R⁴ eine Gruppe der Formeln IIg, IIh oder IIi ist 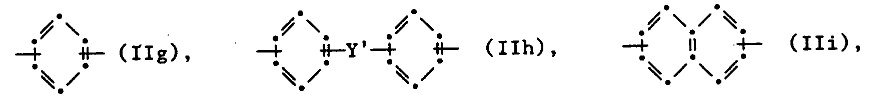 worin Y' eine direkte C-C-Bindung, -O-, -S-, -SO₂-, -C(CF₃)₂- oder -CₙH₂ₙ- ist, n die in Anspruch 1 definierte Bedeutung besitzt, die freien Valenzen im Rest IIg in 1,2- oder 1,4-Position zueinander stehen, im Rest IIh in 1,2- oder 1,4-Position zur jeweiligen Brücke stehen und im Rest IIi in 1,5-, 2,6- oder 2,7-Position zueinander stehen.

7. Verbindungen der Formel I gemäss Anspruch 1, worin X -O- und R⁴ ein Rest der Formel IIj ist 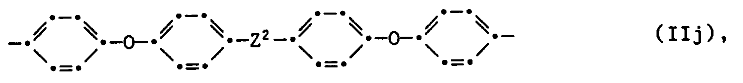 worin Z² die in Anspruch 1 definierte Bedeutung besitzt.

8. Verbindungen der Formel III gemäss Anspruch 1 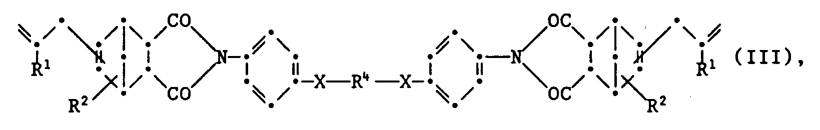 worin R¹, R², R⁴ und X die in Anspruch 1 definierte Bedeutung besitzen.

9. Verbindung der Formel IIIa gemäss Anspruch 8

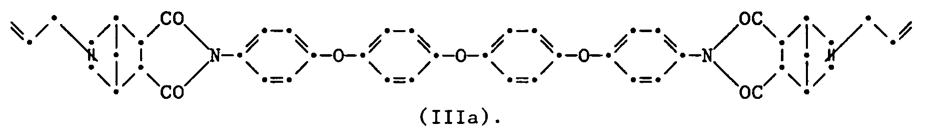

10. Verfahren zur Herstellung der Verbindungen der Formel I gemäss Anspruch 1 umfassend die Umsetzung eines Anhydrids der Formel IVa 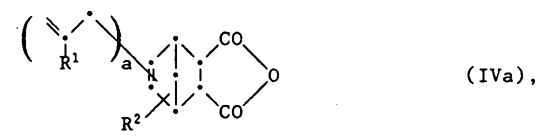 bei erhöhter Temperatur unter Abdestillieren des bei der Reaktion entstehenden Wassers mit einem Diamin der Formel V 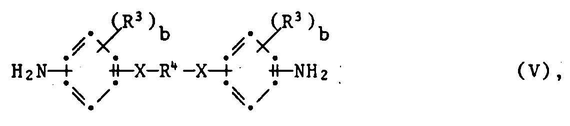 worin R¹, R², R³, R⁴, X, a und b die in Anspruch 1 definierte Bedeutung besitzen.

11. Verfahren zur Herstellung der Verbindungen der Formel I gemäss Anspruch 1, worin X -O- ist, R⁴ ein Rest der Formel IId ist und Y -CO- oder -SO₂- ist, durch Umsetzung von im wesentlichen zwei Aequivalenten eines Phenols der Formel IVb mit einer Verbindung der Formel IVc in Gegenwart einer Base bei erhöhter Temperatur 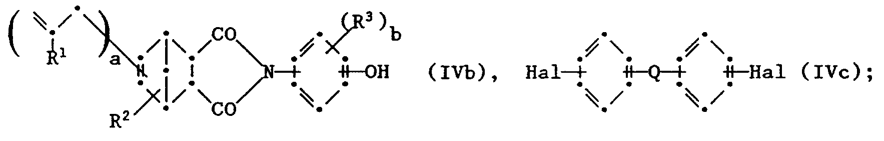 wobei R¹, R², R³, a und b die in Anspruch 1 definierte Bedeutung besitzen, Hal F, Cl oder Br ist und Q -CO- oder -SO₂- ist.

12. Verbindungen der Formeln VIa, VIb, VIc und VId 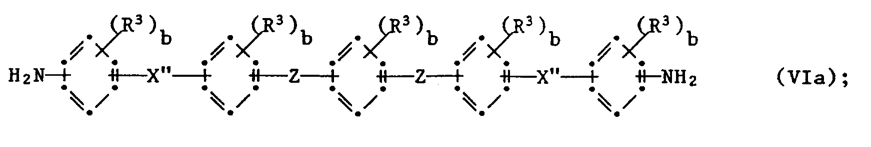 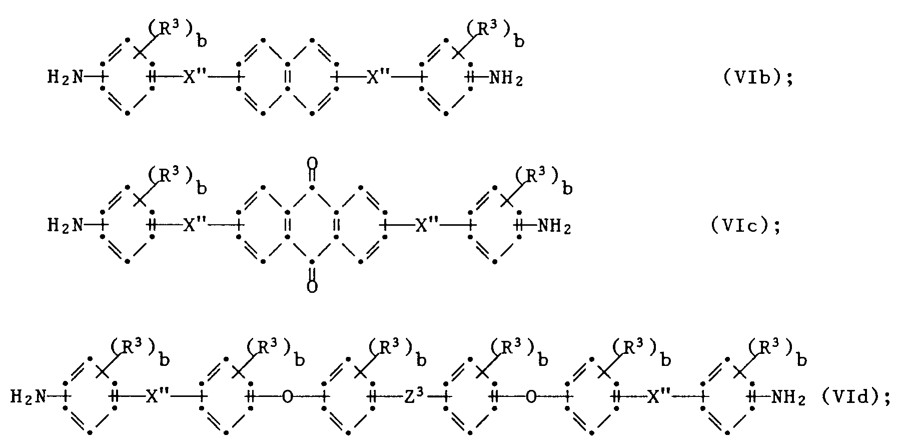 worin R³, Z¹ und b die in Anspruch 1 definierte Bedeutung besitzen, X'' -O-, -S-, -CO- oder -COO- ist und Z³ ist -CO-.

13. Verbindungen der Formeln VIIa, VIIb, VIIc und VIId 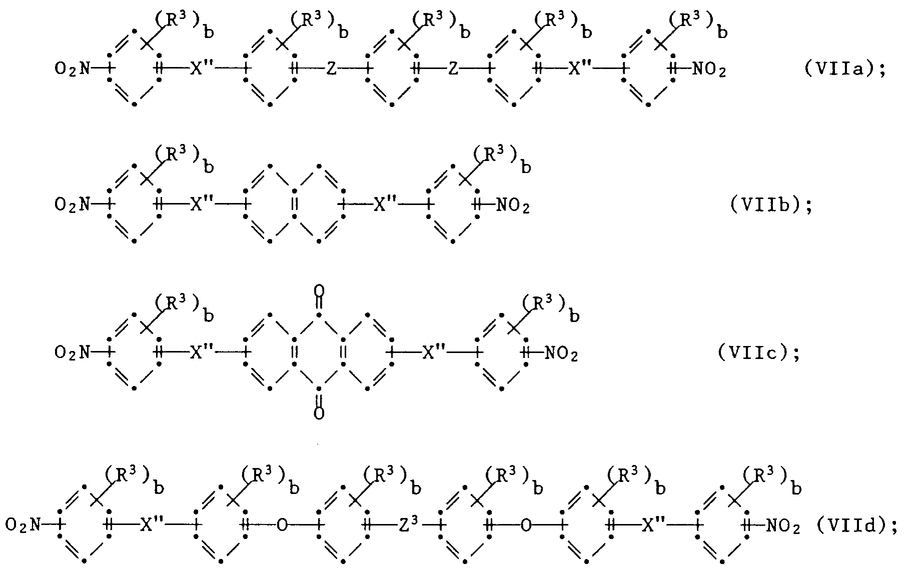 worin R³, Z¹ und b die in Anspruch 1 definierte Bedeutung besitzen und X'' und Z³ die in Anspruch 12 definierte Bedeutung besitzen.

14. Verwendung der Verbindungen der Formel I gemäss Anspruch 1 zur Herstellung von Formkörpern, Beschichtungen oder Verklebungen.

15. Gehärtete Produkte erhältlich durch Erhitzen der Verbindungen der Formel I gemäss Anspruch 1.

## Claims

1. A compound of the formula I 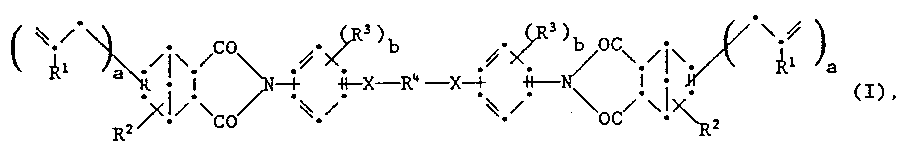 in which a is 1, 2 or 3, b is 0, 1 or 2, R¹ and R², independently of one another, are hydrogen or methyl, R³ is C₁-C₆alkyl, R⁴ is a group of the formula IIa, IIb. IIc, IId, IIe or IIf 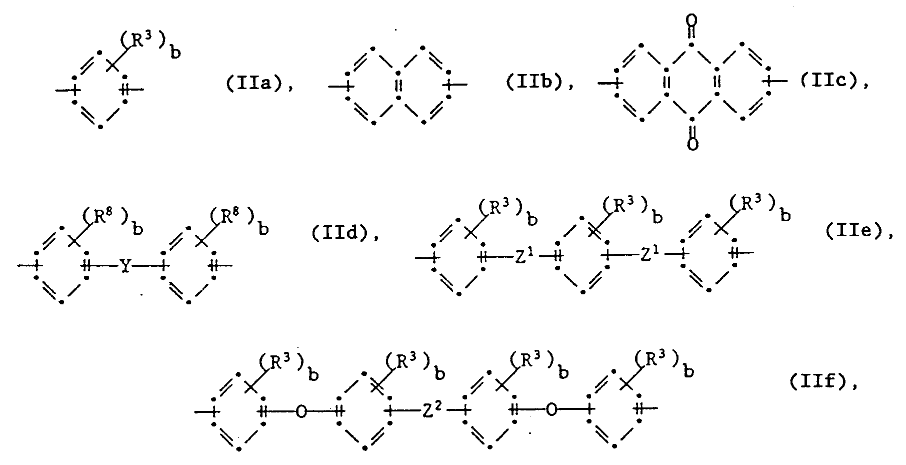 X is -O-, -S- -CO-, -COO-, -CO-NR⁵- or -CR⁶R⁷-, Z¹ is -CR⁶R⁷-, Z² is -SO₂- or -CO-, Y is a direct C-C bond, -O-, -S-, -SO-, -SO₂-, -CO-, -P(O)R⁹-, -COO-, -CO-NR⁵-, -CₙH₂ₙ- or -CR¹⁰R¹¹-, n is an integer from 1 to 12, R⁵ is hydrogen or C₁-C₆alkyl, R⁶ and R⁷, independently of one another, are hydrogen, C₁-C₆alkyl, -CF₃, cyclohexyl or phenyl, or R⁶ and R⁷ together with the common C atom are a cycloalkylidene radical having 5 to 7 ring carbon atoms, R⁸ is C₁-C₆alkyl, chlorine or bromine, R⁹ is methyl, cyclohexyl or phenyl, R¹⁰ is -CF₃, cyclohexyl or phenyl, R¹¹ can adopt one of the meanings of R¹⁰ or is additionally hydrogen, or R¹⁰ and R¹¹ together with the common C atom are a cycloalkylidene radical having 5 to 7 ring carbon atoms.

2. A compound of the formula I according to claim 1, in which a is 1, b is 0 and R² is hydrogen.

3. A compound of the formula I according to claim 1, in which R⁴ is a group of the formula IIa, IIb or IId and b is 0.

4. A compound of the formula I according to claim 1, in which X is -C(CH₃)₂- or in particular -O-.

5. A compound of the formula I according to claim 1, in which Y is a direct C-C bond, -S-, -SO₂-, -CH₂-, -C(CH₃)₂-, -CO- or in particular -O-or -C(CF₃)₂- and in which Z is -CH₂- or -C(CH₃)₂-.

6. A compound of the formula I according to claim 1, in which X is -C(CH₃)₂- or in particular -O- and R⁴ is a group of the formula IIg, IIh or IIi 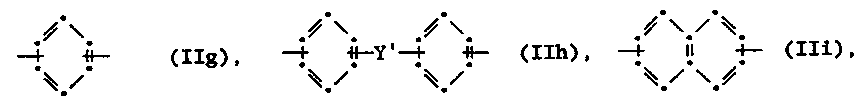 in which Y' is a direct C-C bond, -O-, -S-, -SO₂-, -C(CF₃)₂- or -CₙH₂ₙ-, n is as defined in claim 1, the free valencies are in the 1,2- or 1,4-position with respect to one another in radical IIg, in the 1,2- or 1,4-position with respect to the corresponding bridge in radical IIh and in the 1,5-, 2,6- or 2,7-position with respect to one another in radical IIi.

7. A compound of the formula I according to claim 1, in which X is -O-and R⁴ is a radical of the formula IIj 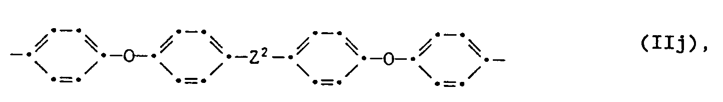 in which Z² is as defined in claim 1.

8. A compound of the formula III according to claim 1 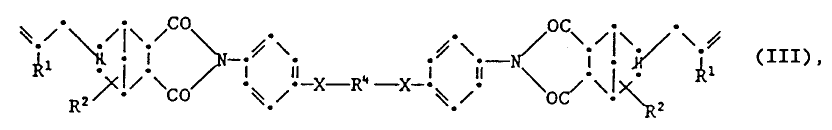 in which R¹, R², R⁴ and X are as defined in claim 1.

9. A compound of the formula IIIa according to claim 8

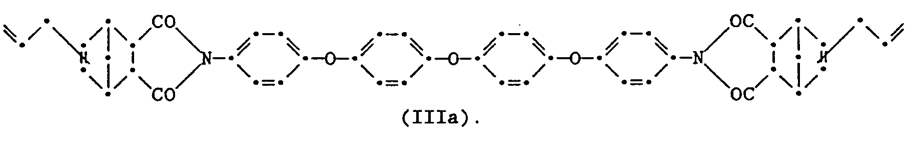

10. A process for the preparation of compounds of the formula I according to claim 1, which comprises reacting an anhydride of the formula IVa 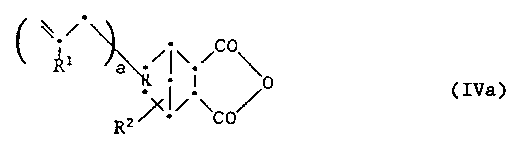 at elevated temperature, while distilling off the water formed in the reaction, with a diamine of the formula V 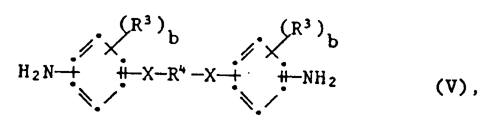 in which R¹, R², R³, R⁴, X, a and b are as defined in claim 1.

11. A process for the preparation of compounds of the formula I according to claim 1, in which X is -O-, R⁴ is a radical of the formula IId and Y is -CO- or -SO₂-, by reaction of essentially two equivalents of a phenol of the formula IVb with a compound of the formula IVc in the presence of a base at elevated temperature 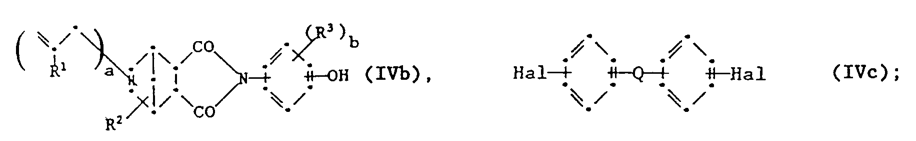 in which R¹, R², R³, a and b are as defined in claim 1, Hal is F, Cl or Br and Q is -CO- or -SO₂-.

12. A compound of the formula VIa, VIb VIc or VId 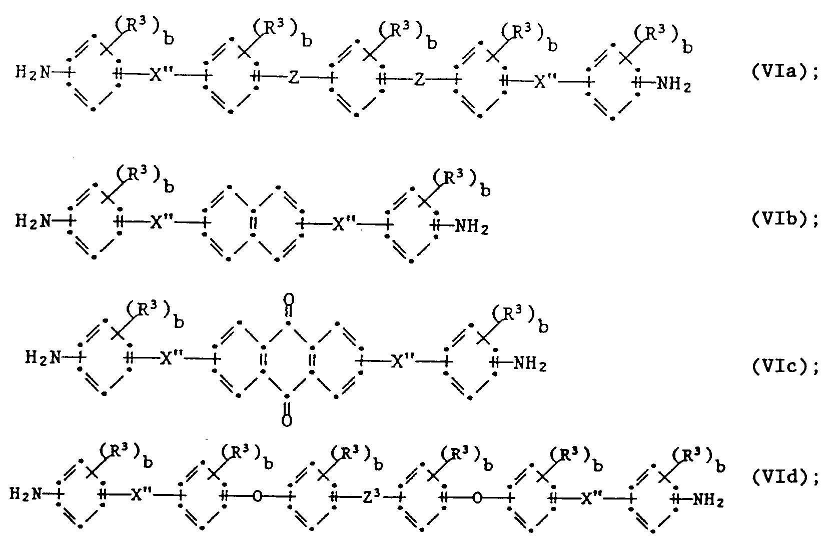 in which R³, Z¹ and b are as defined in claim 1, X'' is -O-, -S-, -CO- or -COO- and Z³ is -CO-.

13. A compound of the formula VIIa, VIIb, VIIc or VIId 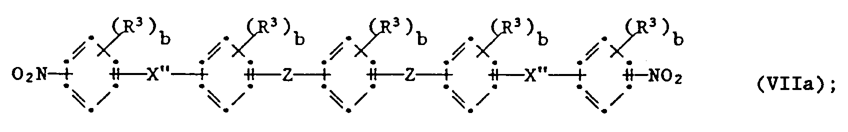 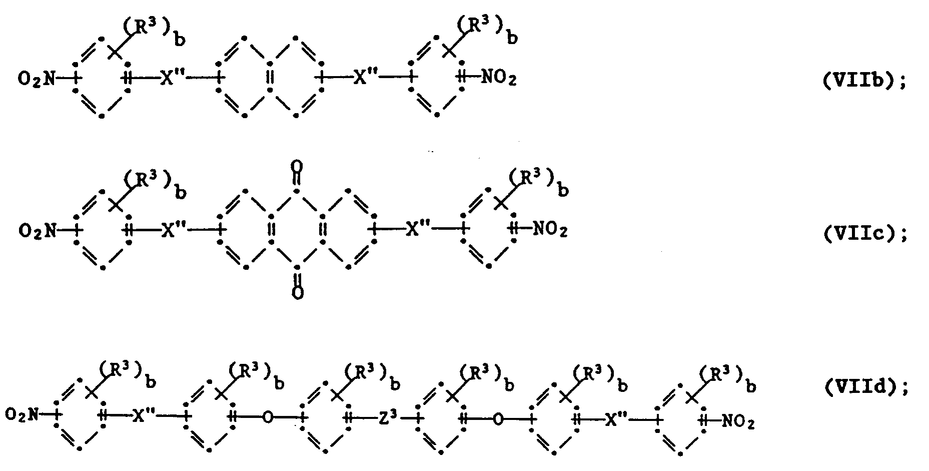 in which R³, Z¹ and b are as defined in claim 1 and X'' and Z³ are as defined in claim 12.

14. Use of a compound of the formula I according to claim 1 for the manufacture of mouldings, coatings or bonded joints.

15. A cured product obtainable by heating a compound of the formula I according to claim 1.

## Revendications

1. Composés de formule I 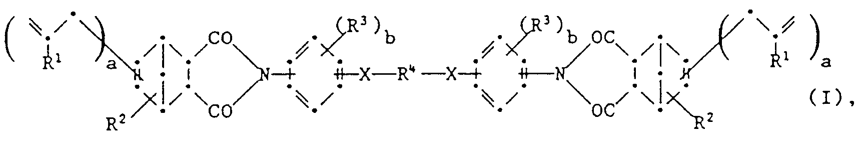 dans laquelle a est 1, 2 ou 3, b est 0, 1 ou 2,
R¹ et R² sont indépendamment l'un de l'autre un hydrogène ou un méthyle, R³ représente un alkyle en C₁-C₆, R⁴ est un groupe de formule IIa, IIb, IIc, IId, IIe ou IIf 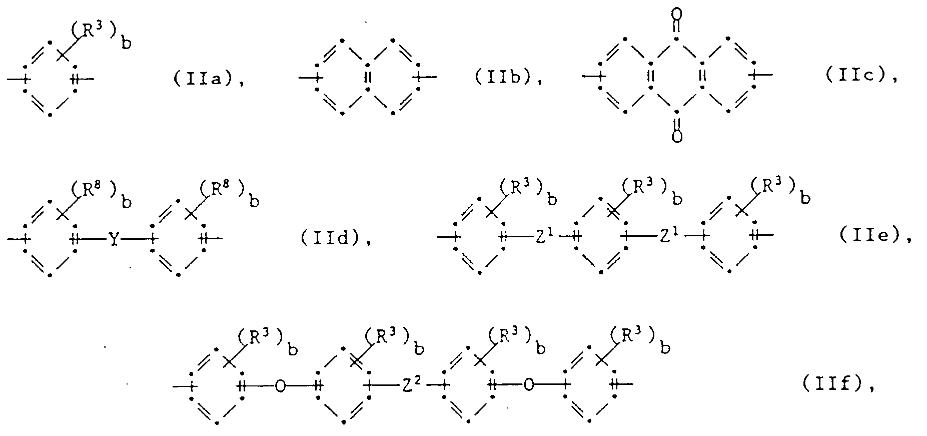 X est -O-, -S-, -CO-, -COO-, -CO-NR⁵- ou -CR⁶R⁷, Z¹ représente -CR⁶R⁷, Z² est -SO₂- ou -CO-, Y est une liaison directe C-C, -O-, -S-, -SO-, -SO₂-, -CO-, -P(O)R⁹-, -COO-, -CO-NR⁵-, CₙH₂ₙ- ou -CR¹⁰R¹¹-, n est un nombre entier de 1 à 12, R⁵ est un hydrogène ou un alkyle en C₁-C₆, R⁶ et R⁷ représentent indépendamment l'un de l'autre un hydrogène, un alkyle en C₁-C₆, -CF₃, un cyclohexyle ou un phényle, ou R⁶ et R⁷ représentent ensemble avec l'atome de carbone commun un reste cycloalkylidène ayant 5 à 7 atomes de carbone cycliques, R⁸ représente un alkyle en C₁-C₆, un chlore ou un brome, R⁹ est un méthyle, un cyclohexyle ou un phényle, R¹⁰ est -CF₃, un cyclohexyle ou un phényle, R¹¹ peut prendre l'une des significations de R¹⁰ ou est en outre l'hydrogène, ou R¹⁰ et R¹¹ représentent ensemble avec l'atome de carbone commun un reste cycloalkylidène ayant 5 à 7 atomes de carbone cycliques.

2. Composés de formule I selon la revendication 1, dans lesquels a est 1, b est 0 et R² est l'hydrogène.

3. Composés de formule I selon la revendication 1, dans lesquels R⁴ est un coupe de formule IIa, IIb ou IId, et b est 0.

4. Composés de formule I selon la revendication 1, dans lesquels X est -C(CH₃)₂- ou en particulier -O-.

5. Composés de formule I selon la revendication 1, dans lesquels Y est une liaison directe -C-C-, -S-, -SO₂-, -CH₂-, -C(CH₃)₂-, -CO-, ou en particulier -O- ou -C(CF₃)₂-, et dans lesquels Z¹ est -CH₂- ou -C(CH₃)₂-.

6. Composés de formule I selon la revendication 1, dans lesquels X est -C(CH₃)₂- ou en particulier -O- et R⁴ est un groupe de formule IIg, IIh ou IIi 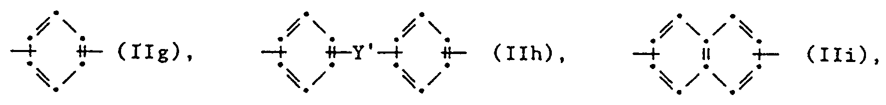 où Y' est une liaison directe C-C, -O-, -S-, -SO₂-, -C(CF₃)₂- ou CₙH₂ₙ-, n a la signification définie dans la revendication 1, les valences libres sont situées dans le reste IIg en position 1,2 ou 1,4 l'une par rapport à l'autre, dans le reste IIh en position 1,2 ou en position 1,4 à chaque fois par rapport au pont, et dans le reste IIi en position 1,5, 2,6 ou 2,7 l'une par rapport à l'autre.

7. Composés de formule I selon la revendication 1, dans lesquels X est -O- et R⁴ est un reste de formule IIj 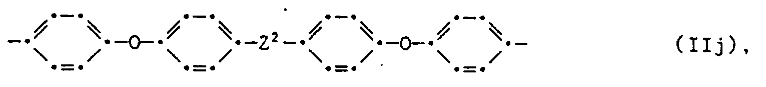 dans laquelle Z² a la signification définie dans la revendication 1.

8. Composés de formule III selon la revendication 1 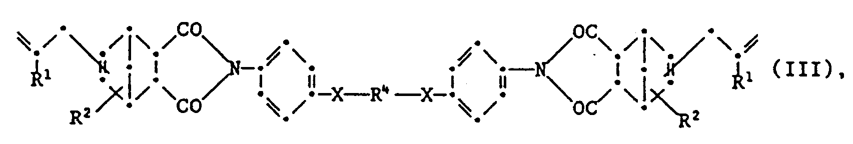 dans laquelle R¹, R², R⁴ et X ont la signification définie dans la revendication 1.

9. Composé de formule IIIa selon la revendication 8

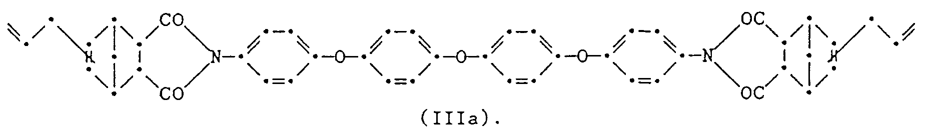

10. Procédé de préparation des composés de formule I selon la revendication 1, comprenant la réaction à température élevée, avec la distillation de l'eau formée pendant la réaction, d'un anhydride de formule IVa 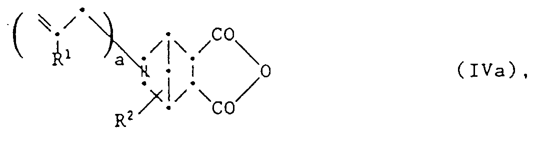 avec une diamine de formule V 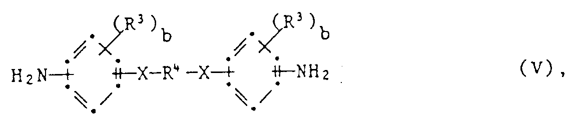 où R¹, R², R³, R⁴, X, a et b ont la signification définie dans la revendication 1.

11. Procédé de préparation des composés de formule I selon la revendication 1 dans lesquels X est -O-, R⁴ est un reste de formule IId et Y est -CO-ou -SO₂-, par réaction à température élevée d'essentiellement deux équivalents d'un phénol de formule IVb avec un composé de formule IVc en présence d'une base: 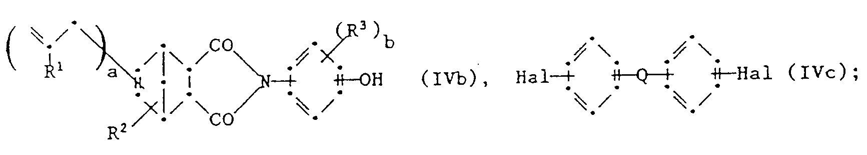 où R¹, R², R³, a et b ont la signification définie dans la revendication 1, Hal est F, Cl ou Br et Q est -CO- ou -SO₂-.

12. Composés de formule VIa, VIb, VIc et VId 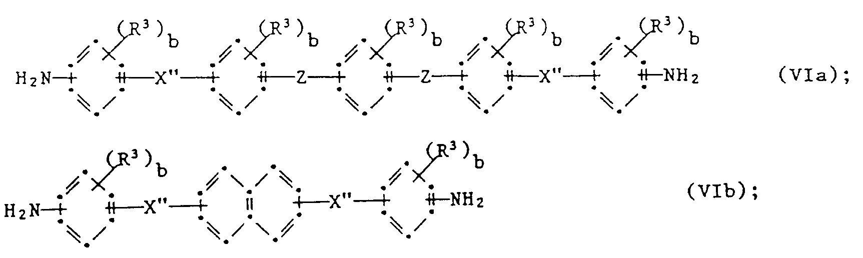 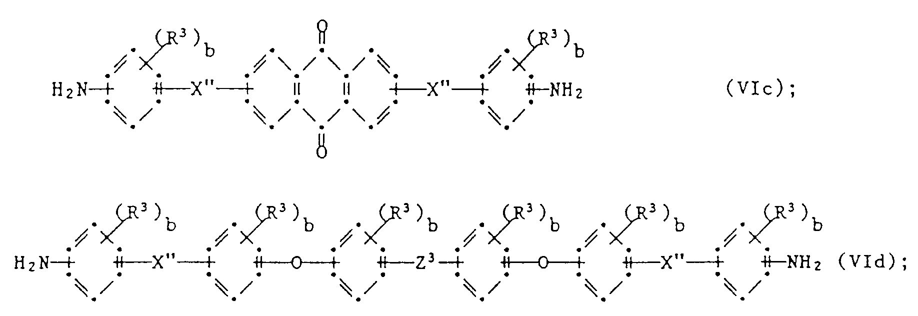 où R³, Z¹ et b ont la signification définie dans la revendication 1, X'' est -O-, -S-, -CO- ou -COO- et Z³ est -CO-.

13. Composés de formule VIIa, VIIb, VIIc et VIId 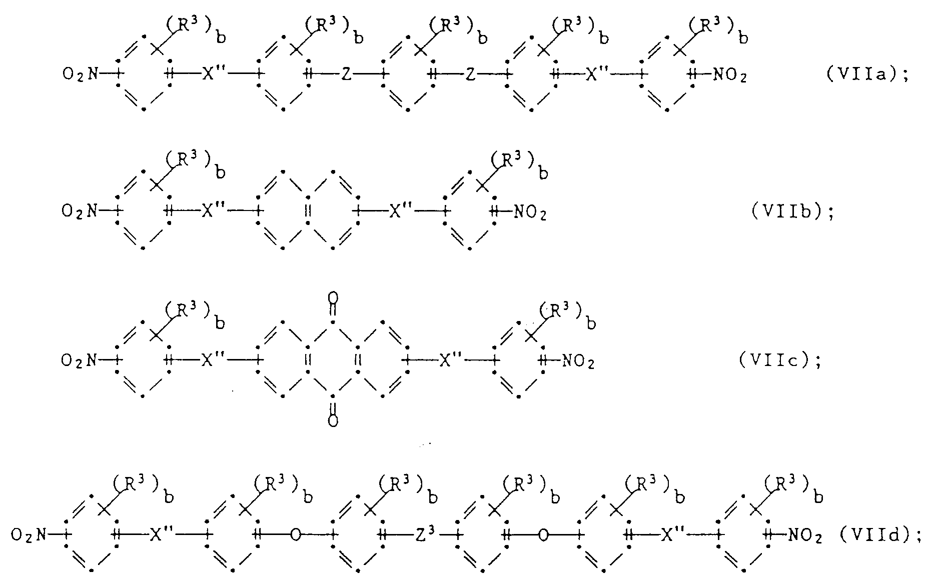 où R³, Z¹ et b ont la signification définie dans la revendication 1 et X'' et Z³ ont la signification définie dans la revendication 12.

14. Utilisation des composés de formule I selon la revendication 1 pour la préparation de corps moulés, de revêtements ou de colles.

15. Produits durcis pouvant être obtenus par chauffage des composés de formule I selon la revendication 1.
